# EUROPEAN PATENT APPLICATION

(11) **EP 4 725 439 A1**
(43) Date of publication of application: **15.04.2026**
(21) Application number: 24206444.2
(22) Date of filing: 14.10.2024
(51) Int. Cl.: A61B 34/20, A61B 34/30, A61B 34/35, A61B 34/00, A61B 17/00

(54) **ROBOTIC SYSTEM WITH CALIBRATION OF AN END-EFFECTOR**

(71) Applicant: Microsure B.V., 5692 EA Son (NL)
(72) Inventor: Boerma, Erik Niels, 5616 BS Eindhoven (NL)
(74) Representative: DTS Patent- und Rechtsanwälte PartmbB

(57) **Abstract**

The present invention provides a method of calibrating an end-effector provided at the distal end of a robot arm, said method comprising: connecting a surgical instrument having a first arm and a second arm to the end-effector, the instrument being adapted for switching between and/or being used in an open configuration and a closed configuration; performing an opening-closing-opening cycle of the surgical instrument; measuring and recording force data related to (closure) force applied to the instrument, especially such as current data and position data during the opening-closing-opening cycle and /or related sensor data; defining and recording a relationship between force data and position data during the opening-closing-opening cycle; analyzing the relationship and determining a closing point of the surgical instrument, and calibrating the end-effector based on the determined closing point of the surgical instrument.

## Description

The present invention belongs to the technical field of robotic systems and methods for use in surgical, microsurgical or super-microsurgical procedures.

In particular, the present invention refers to a method of calibrating an end-effector, provided at the distal end of a robot arm, after a surgical instrument having a first arm and a second arm is connected to the end-effector.

For instance, said surgical instrument can be a hinged surgical instrument (i.e. a surgical instrument that comprises at least one hinge).

Examples of hinged instruments for use in surgical, microsurgical or super-microsurgical procedures include scissors-type or forceps-type instruments, dilators, or the like.

The invention further refers to a robotic system comprising, inter alia, a processing means adapted to run a dedicated non-volatile computer-readable medium to carry out the above-mentioned method.

The robotic system is adapted for use in surgery, microsurgery or super-microsurgery procedures.

As a non-limiting example, the robotic system may be adapted to perform anastomoses.

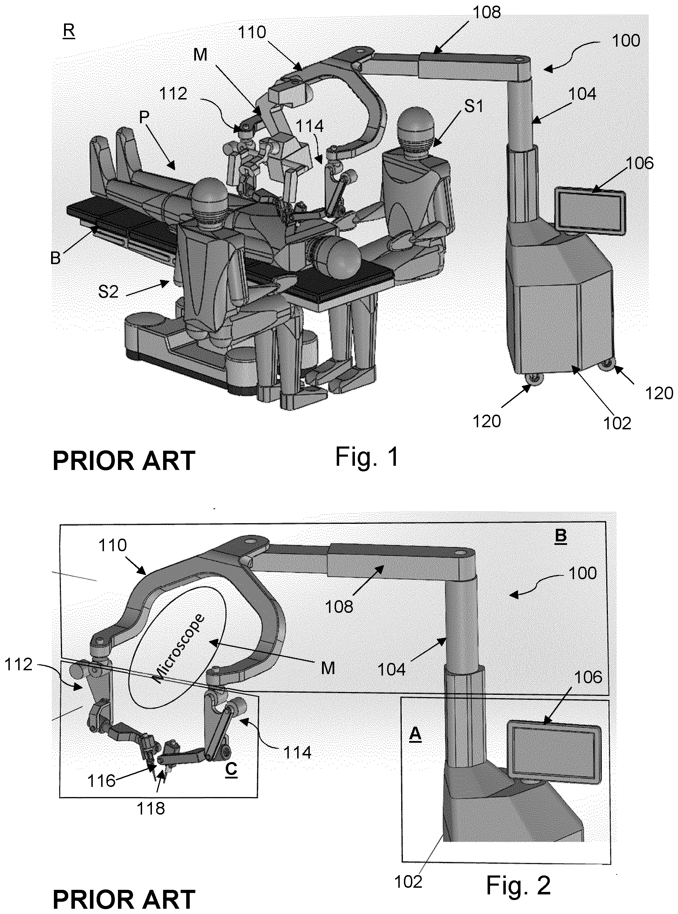

An exemplary configuration of a robotic system for use in surgery, microsurgery or super-microsurgery is shown in **Figs. 1-2****.**

The invention further refers to a computer-readable non-volatile storage medium comprising computer-readable instructions that, when executed by a processing means, causes said processing means to carry out the above-mentioned method.

Surgical instruments are often configured to be manually held and manipulated by an operator (e.g., a surgeon).

Alternatively, said surgical instruments can be manipulated through a robotic system comprising one or more robot arms (two in the configuration of **Figs. 1-2**).

The use of a robotic system facilitates accurate movements with micrometer precision without substantial tremor, thereby superseding the limitations of human hand manipulation.

Robotic manipulation might require the use of an adapter, providing an interface between the end-effector and a surgical instrument to be manipulated through the robot arm.

Surgical instrument adapters of this kind are known in the art.

EP3363401A1, filed in the name of the same applicant, discloses a robotic manipulator interface for coupling a hinged surgical tool to a manipulator of a surgical robot, the interface comprising a first interface member to be coupled to the manipulator, and a second interface member to be coupled to the first interface member, the second interface member being arranged to mount the hinged surgical tool, wherein the first interface member comprises a pinching mechanism for pinching the hinged surgical tool when the second interface member is coupled to the first interface member.

EP1232836A1 discloses a robot carrier for tweezers, said robot carrier comprising a housing to be mounted on the arm of the robot and enclosing an operating drive for tweezers and a mounting and indexing support. A motor actuates pusher cam arms to engage the legs of the tweezers to open and close them. The housing is open at one end to receive the tweezers.

WO2022233585A1, filed in the name of the same applicant, discloses an improved end-effector system for a surgical, microsurgical or super-microsurgical robot arm composing an end-effector including an end effector base, a first movable finger and a second movable finger, said first and second movable fingers extending from the end-effector base; a sterile drape, configured to cover the end-effector; and a surgical instrument adapter, configured to slide onto the draped first and second fingers of the end-effector and be locked in place by means of a locking mechanism.

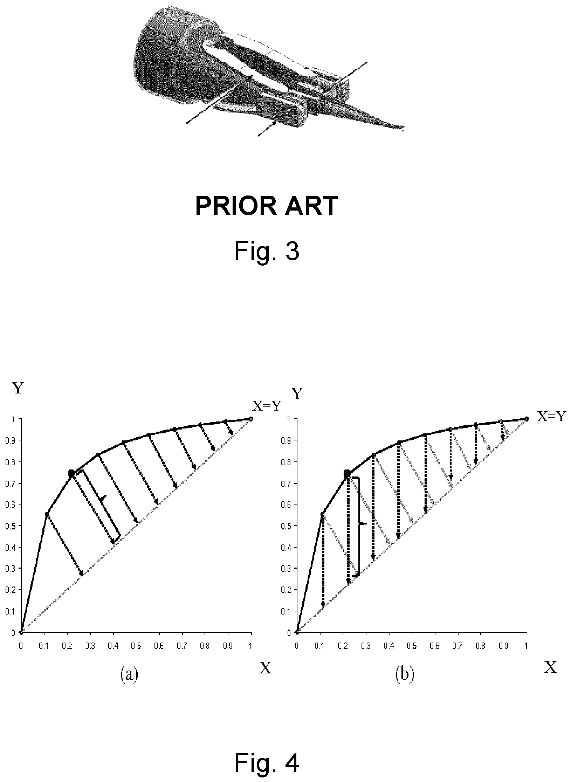

An example of this known end-effector system is shown in **Fig. 3****.**

Surgical instruments of the same kind, even from the same manufacturer, may have some small structural differences between one another, especially when hand-finished or when some slight constructional changes are carried out by the manufacturer.

The use of the instrument, e.g. wear, and/or cleaning operations for re-use, handling, transportation, and/or re-packaging may also induce some shape variations of the instrument.

When the instrument is manually operated, these shape variations are almost irrelevant, as the surgeon will simply adapt to any variation based on tactile feedback during use.

However, when the instrument is robotically handled and manipulated, controlled by an input device, tactile feedback is hampered, if present at all.

For instance, required forces may not be reached, or maximum forces may be exceeded. Similarly, opening of the instrument, e.g. a scissors-type or a forceps-type instrument, may be either too little or too much.

This increases the risk of tissue damage during surgery.

Also, the obtained surgical outcome may be suboptimal.

The (possible) adapter may comprise a portion that is adapted for connection to the robot, and a portion that is adapted to accommodate any type of surgical instrument.

While the portion that is adapted for connection to the robot remains the same for all kind of adapters, the portion that is adapted for connection to the instrument varies depending on the type of instrument to be used to carry out a desired procedure.

Variation in design and production of the adapter may add further variation in the instrument itself.

Still further, to maintain the sterile barrier during surgery, a sterile drape is used to cover the robot arm and the end-effector. The adapter and the instrument are mounted on this sterile drape.

The presence of the drape adds further variation both in the adapter and the instrument.

In particular, upon placing the instrument in the adapter, and further placing the adapter over the drape covering the end-effector, additional variation may occur.

In principle, instruments that are adapted for manual operation do not have mechanical positioning geometry intended for use with a robotic device.

Accordingly, their placement with respect to the robot arm (through the adapter) is repeatable within a range of positions.

Since there is flexibility in the adapter and drape, some variation may be introduced in instrument positioning within the end-effector.

By the nature of the above-described variations, cumulative variations are different every time a surgical instrument is connected to the end-effector. Even when an instrument is replaced and re-attached in the context of the same surgical, microsurgical or super-microsurgical procedure, variation may still be present.

Accordingly, there is the need to provide an improved solution allowing addressing the above-listed drawbacks occurring when a surgical instrument, especially an instrument designed for manual operation, is connected to a draped end-effector.

In particular, there is the need for a solution allowing obtaining precise and reliable robotic manipulation when operating the instrument, e.g. a hinged instrument designed for manual operation, thereby preventing tissue damage and optimizing surgical outcome.

In view of the above, it is an object of the invention to provide a method of calibrating an end-effector allowing easily and reliably calibrating the end-effector when a surgical instrument, e.g. a hinged surgical instrument, is connected thereto, thus preventing the occurrence of tissue damage and improving surgical outcome.

The above-specified object is achieved by the provision of a method of calibrating an end-effector as defined in claim 1.

According to the invention, a method of calibrating an end-effector provided at the distal end of a robot arm comprises:
connecting a surgical instrument having a first arm and a second arm to the end-effector, the instrument being adapted for switching between and/or being used in an open configuration and a closed configuration;
performing an opening-closing-opening cycle of the surgical instrument;
measuring and recording force data related to (closure) force applied to the instrument, especially such as current data and position data during the opening-closing-opening cycle and /or related sensor data;
defining and recording a relationship between force data and position data during the opening-closing-opening cycle;
analyzing the relationship and determining a closing point of the surgical instrument, and
calibrating the end-effector based on the determined closing point of the surgical instrument.

The present invention provides a method of calibrating an end-effector.

The end-effector is provided at the distal end of a robot arm.

Calibration is required whenever an instrument is connected or re-connected to the end-effector.

The robot arm may be adapted for use in a robotic system for use in surgery, microsurgery or super-microsurgery.

For instance, the robotic system may be adapted to perform anastomoses.

The robot arm and the end-effector are draped to maintain the sterile barrier during surgery.

The method comprises connecting a surgical instrument to the end-effector.

In particular, the surgical instrument has a first arm and a second arm.

The instrument has a certain first stiffness in the open state and a second certain stiffness in the closed state (either closed without an object like a needle inbetween or gripping an element like needle, resulting in both cases with the second certain stiffness, which is larger than the first certain stiffness), which has to be taken into account and is taken into account as part of the system and method according to the present disclosure/invention.

In particular, the instrument is adapted for switching between an and/or being used in an open configuration and a closed configuration.

As a non-limiting example, the surgical instrument may be a hinged surgical instrument.

For instance, the instrument may be a scissors-type or forceps-type instrument.

Instruments of this kind are operated by being brought from an open configuration to a closed configuration.

As a further example, the instrument may be a dilator.

A dilator is also able to switch between an open configuration and a closed configuration.

However, a dilator is operated by being brought from a closed configuration to an open configuration.

A similar operation criterion characterizes scissors-type instruments having their cutting portion formed on the external side of each blade, configured to cut a target tissue upon being brought from a closed configuration to an open configuration.

The method further comprises performing an opening-closing-opening cycle of the surgical instrument.

The method further comprises measuring and recording force data related to (closure) force applied to the instrument, especially such as current data and position data during the opening-closing-opening cycle and /or related sensor data.

As a non-limiting example, said current component data may be quadrature current component data.

The definition "quadrature current component" denotes a component of motor current that is orthogonal to a direct current component.

This aspect is crucial in order to understand motor torque which, in turn, is represented by the value of the quadrature current.

The definition "closure point" denotes the moment when the instrument reaches a close configuration.

Here the change between the first and second certain stiffness occurs.

If so, the method can be as follows:
The method of calibrating an end-effector provided at the distal end of a robot arm comprises:
connecting a surgical instrument having a first arm and a second arm to the end-effector, the instrument being adapted for switching between and/or being used in an open configuration and a closed configuration;
performing an opening-closing-opening cycle of the surgical instrument;
measuring and recording force data related to (closure) force applied to the instrument, especially such as current data, here quadrature current data, and position data during the opening-closing-opening cycle and /or related sensor data;
defining and recording a relationship between force data by using the quadrature current component and position data during the opening-closing-opening cycle; so defining and recording a relationship between quadrature current component and position data during the opening-closing-opening cycle;
analyzing the relationship and determining a closing point of the surgical instrument by
defining a data curve in a e.g. Cartesian coordinate system (or any other suitable coordinate system), showing a relationship between position data on a first dimension of the coordinate system (the X-axis) and quadrature current component data on a second dimension of the coordinate system (the Y-axis), during the opening-closing-opening cycle;
   analyzing an interrelation between the first dimension and the second dimension of the defined data curve to detect the moment where the quadrature current component starts deviating upwards, corresponding to the point where the surgical instrument reaches the closed state, and
   calibrating the end-effector based on the determined closing point of the surgical instrument.

The method further comprises defining and recording a relationship between current component data and position data, during the opening-closing-opening cycle.

The method further comprises defining a data curve in a coordinate system, showing the relationship between position data on a first dimension of the coordinate system and current component data on a second dimension of the coordinate system, during the opening-closing-opening cycle.

As a non-limiting example, said coordinate system can be a Cartesian coordinate system.

The method further comprises analyzing an interrelation between the first dimension and the second dimension to detect the moment where the current component starts deviating upwards, corresponding to the point where the surgical instrument reaches the closed state.

That is, the data curve is accurately analyzed to pinpoint the exact moment where the current component starts deviating upwards, this configuring the closure point of the surgical instrument connected to the end-effector.

One possible example and option to analyze the data curve could be, e.g., an analysis of the gradient of the curve. E.g., by reaching a certain threshold value or by maintaining a certain gradient for a specific amount of time this could be interpreted as having arrived at the closing point of the instrument (i.e., the change from the first certain stiffness to the second certain stiffness). The analysis may include alternatively or additionally an interpretation of the first derivative of the data curve (describing the gradient of the data curve).

In the method of the invention, the closure point of the surgical instrument is defined without the use of a force sensor or the like.

The method further comprises calibrating the end-effector based on the determined closing point of the surgical instrument.

The invention is based on the basic idea that, by calibrating the end-effector based on the closure point of a surgical instrument connected thereto, precise, accurate, and reliable manipulation of the instrument can be implemented, even when the instrument is designed for manual operation and regardless of any variation. This allows preventing the occurrence of tissue damage during surgery. Also, the surgical outcome is significantly improved.

The calibration method of the invention allows manipulating the surgical instrument with improved accuracy when compared to manual operation.

Also, potential issues relating to deviations of any kind, either due to a slight change in the instrument shape or to any other reason as discussed in detail in the foregoing, can be easily addressed even in the absence of a haptic feedback.

Advantageously, the method may further comprise a step of noise reduction.

Said step of noise reduction may consist of or comprise smoothing the measured current component data.

This allows reducing noise, e.g. due to cogging, friction, or the like.

Accordingly, the overall level of accuracy can be further increased.

Preferably, said step of noise reduction is implemented by using a moving average.

According to a first embodiment, the method of the invention further comprises normalizing the smoothed current component data within a unit square.

This allows standardizing analysis scale.

Here, the method further comprises the steps of:
defining a data vector in the data curve and rotating the defined vector by 45° to align a slope of the axes of the coordinate system, with X=Y with the curve, this enabling calculating a distance;
calculating an amplitude of the rotated vector (|V|), this value corresponding to a difference (X-Y);
identifying a maximum amplitude of the difference (X-Y), corresponding to the closure point of the surgical instrument, and
scaling data back to the original dimensions to locate the closure point within the actual dataset.

Accordingly, the end effector can be calibrated with a high level of precision and reliability.

Although this approach has proven effective in testing and evaluation phases, nevertheless its computational complexity and processing time precludes its use in production environments.

In particular, production environments are characterized by strict time constraints.

These time constraints, especially the requirement to operate within a single EtherCAT cycle (maximally 250 microseconds), posed a significant challenge.

A first attempt carried out by the inventors, trying to directly translate refined offline process into C++ for real-time application, resulted in execution times around 2 milliseconds, significantly exceeding the operational threshold required for efficient production calibration.

To solve the issue, a method according to the second embodiment of the invention is provided, comprising:
defining an angle of rotation and rotating the data curve according to the defined angle of rotation, such that a slope from a minimum curve point to a maximum curve point is aligned with the axis of the first dimension, and
calculating a maximum amplitude of the rotated data curve to identify the closure point of the surgical instrument.

The aforementioned steps can be implemented directly after smoothing of the measured current component data, without any additional normalization step.

This simplified approach allows accomplishing calibration of the end-effector in approximately 120 microseconds, thus largely within the time constraints characterizing production environments.

Summarizing, this simplified approach provides an optimized process, meeting the tight timing requirements characterizing production environment, at the same time without jeopardizing the overall standards of accuracy and reliability.

The method of the invention may further comprise mapping a movement range for a manipulator provided on a surgeon side unit of a robotic system for use in surgery, microsurgery, or super-microsurgery, to control operation of the end-effector based on the recorded current component data and position data, and the defined closure point of the instrument.

According to a non-limiting example, said manipulator is a joystick.

A range of movement of the manipulator, e.g. joystick stroke, can be adjusted based on the features of the instrument to be operated through the robot arm.

For instance, the manipulator can be mapped to ensure smooth and controlled closing of a scissors-type instrument.

In principle, a predefined mapping for the manipulator, e.g. a joystick, may as well be reversed to obtain a negative mapping.

This can be implemented for use with an instrument such as a dilator, to accomplish a smooth opening (instead of a smooth closure).

Advantageously, the position data for the surgical instrument are based on encoder data.

This allows defining position data with great accuracy and reliability.

Advantageously, the step of calibrating the end-effector may comprise defining, based on the determined closing point of the instrument, one or more among the following operation parameters for the instrument:
maximum opening position, and/or
maximum closure position, and/or
maximum closure force.

Accordingly, the instrument can be smoothly manipulated with high level of accuracy, preventing tissue damage.

Also, the surgical outcome can be optimized.

The invention further refers to a robotic system for use in surgery, microsurgery or super-microsurgery.

For instance, the system may be adapted to perform anastomoses.

The system comprises at least one robot arm.

The system further comprises an end-effector, provided at the distal end of the at least one robot arm.

There is also a sterile drape provided in the system, said sterile drape being configured to cover the at least one robot arm and the end-effector, to maintain the sterile barrier during surgery.

The system further comprises at least one surgical instrument having a first arm and a second arm.

The at least one surgical instrument is adapted for switching between an open configuration and a closed configuration.

As a non-limiting example, the at least one instrument may be a hinged surgical instrument.

For instance, the at least one surgical instrument can be a scissors-type or a forceps-type instrument.

As a further example, the at least one surgical instrument can be a dilator.

The system further comprises a surgical instrument adapter, providing an interface for operative connection between the at least one surgical instrument and the end effector.

Still further, the system comprises a processing means that is configured and adapted to run a dedicated non-volatile computer-readable medium to implement and/or process and/or perform the above-defined method steps.

Still further, the invention refers to a computer-readable non-volatile storage medium comprising computer-readable instructions that, when executed by a processing means, causes said processing means to implement and/or process and/or perform the above-defined method steps.

Further advantages of the present invention shall now be disclosed in connection with the drawings, where:
- **Fig. 1**: schematically shows a robotic system for use in surgery, microsurgery or super-microsurgery according to the prior art, in an operative state;
- **Fig. 2**: schematically shows a detail of the surgical robotic system of **Fig. 1****,** where different sections of the robotic system, respectively denoted with letters **A, B, C,** are identified;
- **Fig. 3**: shows an end-effector system provided with a surgical instrument adapter, according to the prior art. Here, a surgical instrument is mounted to the adapter;
- **Fig. 4**: is a diagram showing data curve normalization and vector rotation in a coordinate system, according to an embodiment of the invention. In detail: **a)** data curve depicting the relationship between position data on a first dimension of the coordinate system (X-axis) and current component data on a second dimension of the coordinate system (Y-axis) during an opening-closing-opening cycle of a surgical instrument, e.g. a hinged surgical instrument, after normalization. A data vector is defined in the data curve; **b)** normalized curve as depicted in **a),** but after rotation of the data vector by 45°;
- **Fig. 5**: is a diagram showing the results of an experimental implementation involving 10 exemplary opening-closing-opening cycles. In detail: the first plot represents Iq(t) (i.e., quadrature current (t)); the second plot represents Pos(t) (i.e., position (t)); and the third plot represents Iq(pos) (i.e., quadrature current depending on position).
- **Fig. 6**: is a diagram showing normalized curve, amplitude of the rotated vectors, and detected closure point, in the experimental implementation of **Fig. 5****;**
- **Fig. 7**: is a diagram showing a defined data curve according to another embodiment of the invention and its rotation by a predefined angle α, such that the slope from a minimum curve point to a maximum curve point is aligned with the axis of the first dimension (X-axis).

**Fig. 1** shows an example of a robotic system for use in surgery, microsurgery or super-microsurgery.

Said exemplary system is denoted with 100.

Here, the system 100 is shown in an operating state.

A first surgeon S1 and a second surgeon S2 are located at a respective side of an operating bed B to perform a surgical, microsurgical or super-microsurgical operation on a patient P, laying on operating bed B.

The robotic system 100 includes a base station 102.

The surgical robotic system 100 further includes a base column 104, here in the shape of a cylindrical pillar.

In the shown example, the base station 102 conveniently includes a display module 106 for displaying information to one or more operators, e.g. the surgeons S1, S2 or other personnel that is present in the operating room R.

Also, the display module 106 may be used to define a user interface allowing one or more operators to provide a user input.

The base station 102 comprises a plurality of wheels 120 for ease of placement of the robotic system 100 at a desired location within the operating room R.

In the shown example, the robotic system 100 is equipped with a suspension arm 108, a fork-like element 110, a first surgical, microsurgical or super-microsurgical robot arm 112, and a second surgical, microsurgical or super-microsurgical robot arm 114.

Here, the suspension arm 108 is horizontally arranged.

Also, the suspension arm 108 has two parts allowing to adjust its length by telescopically moving said parts relatively to each other.

It is also possible that a different means is used for length adjustment of the suspension arm 108, e.g. a SCARA-mechanism, hinge mechanisms, or the like.

The suspension arm 108 is connected to the base column 104.

The suspension arm 108 carries the fork-like element 110 (e.g. a bracket).

As shown in detail in **Fig. 2****,** the fork-like element 110 carries the first surgical, microsurgical or super-microsurgical robot arm 112 and the second surgical, microsurgical or super-microsurgical robot arm 114.

Each of the first and second robot arms 112, 114 is connected to an end-effector system carrying a surgical instrument 116, 118 (**Fig. 2**).

The surgical instruments 116, 118 may be of the same kind, or may be different from each other.

The suspension arm 108 is configured to rotate about the longitudinal axis of the base column 104, to allow desired positioning of the fork-like element 110 and the robot arms 112, 114 with respect to the surgical site.

Similarly, the fork-like element 110 is configured to rotate about an axis of the suspension arm 108, i.e. parallel to the longitudinal axis of the base column 104. The robotic system 100 may be adapted for use with a conventional microscope M, as shown in **Fig. 2****.**

Alternatively, a fully digital microscope (not shown) or a so-called hybrid microscope (not shown) can be used. In such a case, one or more surgeons will be located at a separate console, and a robot trolley will be located at the position of one of the surgeons S1, S2 shown in **Fig. 1****.**

The surgical robotic system 100 provides a large patient side setup, this meaning that no assembly is required after draping.

**Fig. 2** shows different sections A, B, C of the surgical robotic system 100.

In section A, there is no movement and everything is thus stationary.

The display means 106 (included in section A) can be activated by one or more operators.

In section B, there is some movement during the surgery, in order to correctly reposition the surgical instruments 116, 118 over the patient P.

As shown in **Fig. 2****,** a space is defined between the surgical robotic arms 112, 114 for a microscope (not illustrated but only symbolized in **Fig. 2**).

Section C includes, inter alia, the end-effector.

Here, several movements occur in the course of the surgical procedure.

In this context, the functions of the end-effector are, inter alia, to actuate the surgical instruments 116, 118, provide a mounting interface, and rotate around the instrument axis.

In the following, a method of calibrating an end-effector according to the invention will be described in detail.

The end-effector is provided at the distal end of a robot arm.

The robot arm may be of the same kind of the first and second robot arms 112, 114 described above with reference to **Figs. 1-2****.**

The robot arm can be adapted for use in a surgical, microsurgical or super-microsurgical robotic system, such as the system 100 described above with reference to **Fig. 1****.**

The robot arm and the end-effector are draped to ensure the sterile barrier when a surgical, microsurgical or super-microsurgical procedure is carried out.

In particular, the method comprises the steps of:
connecting a surgical instrument having a first arm and a second arm to the end-effector, the instrument being adapted for switching between and/or being used in an open configuration and a closed configuration;
performing an opening-closing-opening cycle of the surgical instrument;
measuring and recording force data related to (closure) force applied to the instrument, especially such as current data and position data during the opening-closing-opening cycle and /or related sensor data;
defining and recording a relationship between force data and position data during the opening-closing-opening cycle;
analyzing the relationship and determining a closing point of the surgical instrument, and
calibrating the end-effector based on the determined closing point of the surgical instrument.

As a non-limiting example, the surgical instrument can be a hinged surgical instrument.

For instance, the surgical instrument can be a scissors-type or a forceps-type instrument, operated by being brought from an open configuration toward a closed configuration.

Alternatively, said surgical instrument can be a dilator, which is conversely operated by being brought from a closed configuration toward an open configuration. The same principle applies when a scissor-type instrument is used, where cutting portions of the scissors are provided on an external side of each blade.

By calibrating the end-effector based on the closure point of a surgical instrument connected thereto, precise, accurate, and reliable manipulation of the instrument can be implemented, even when the instrument is designed for manual operation and/or regardless of any variation.

Accordingly, the risk of tissue damage can be largely prevented, and the surgical outcome can be significantly enhanced.

Preferably, the position data for the surgical instrument are based on encoder data.

As a non-limiting example, the coordinate system can be a Cartesian coordinate system.

As a non-limiting example, the current component data can be quadrature current component data.

Preferably, the step of calibrating the end-effector may comprise defining, based on the determined closing point of the surgical instrument, one or more among the following operation parameters for the instrument:
maximum opening position, and/or
maximum closure position; and/or
maximum closure force.

Accordingly, the occurrence of tissue damage during surgery, e.g. for the applied forces exceeding the maximum, can be prevented.

Also, by calibrating the end-effector so that the surgical instrument is operated with the required forces and according to an optimized maximum opening, the surgical outcome is enhanced.

A calibration method according to a first embodiment of the invention will be described in the following.

Here, the method further comprises a step of noise reduction.

In particular, said step of noise reduction may consist of or comprise smoothing the measured current component data, e.g. quadrature current component data, to reduce noise (e.g. due to cogging, friction, or the like).

Preferably, this step is implemented by using a moving average.

The method further comprises normalizing smoothed current component data within a unit square, to standardize analysis scale.

An example of normalized data curve according to the embodiment is shown in **Fig. 4(a)****.**

A data vector is defined in the data curve, as illustrated in the same **Fig. 4(a)****.**

Then, the defined data vector is rotated by 45° as depicted in **Fig. 4(b)****,** to align a slope of the axes of the coordinate system, with X=Y with the curve.

An amplitude of the rotated vector (|V|) is then calculated, this value corresponding to a difference (X-Y).

Subsequently, a maximum amplitude of the difference (X-Y) is identified, this value corresponding to the closure point of the instrument.

Eventually, data are scaled back to the original dimensions to locate the closure point within the actual dataset.

The end-effector is then calibrated based on the identified closure point of the surgical instrument.

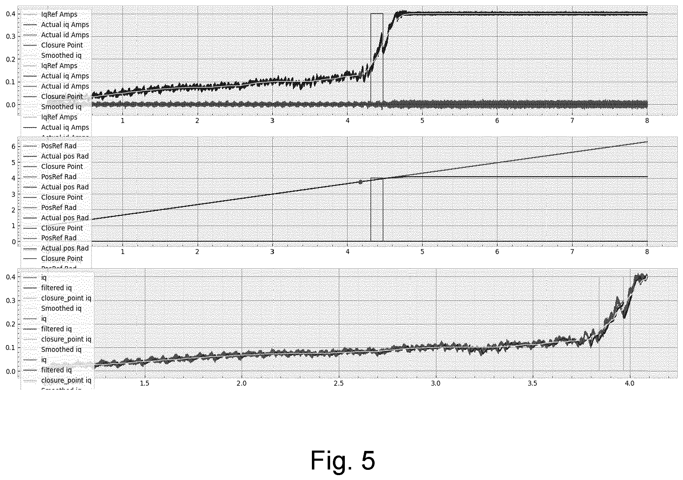

**Fig. 5** shows an experimental implementation involving 10 opening-closing-opening cycles of a surgical instrument.

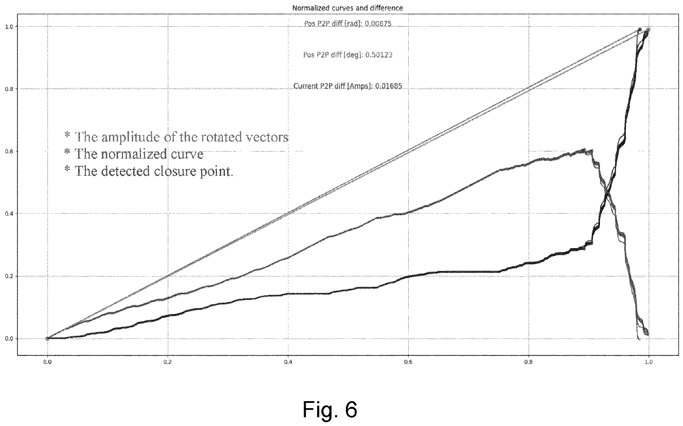

**Fig. 6** shows the normalized data curve, the amplitude of the rotated vectors, as well as the detected closure point, in the experimental implementation of **Fig. 5****.**

The calibration method according to the embodiment has proven effective for the purpose of testing and validation.

Nevertheless, its computational complexity, as well as its processing time, preclude its use in production environments, which are characterized by significant time constraints (e.g., a maximum of 250 microseconds when it is required to operate within a single EtherCAT cycle).

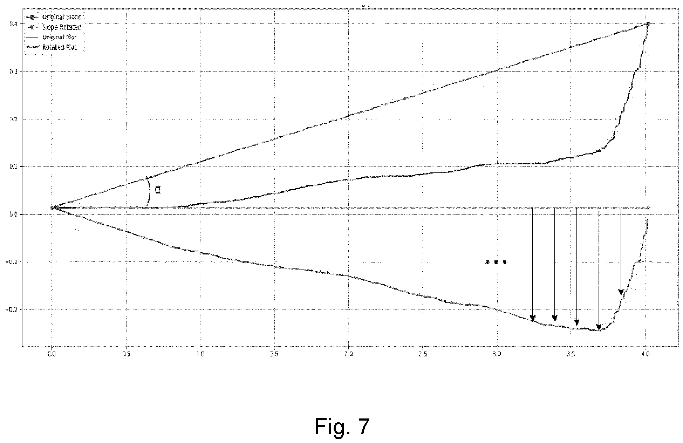

For this purpose, a simplified calibration process according to a second embodiment of the invention is provided (**Fig. 7**).

In this embodiment, the method may as well comprise a noise reduction step consisting of or comprising smoothing the measured current component data, e.g. by using a moving average, for noise reduction.

However, normalization of the smoothed current component data within a unit square is not required.

Here, an angle of rotation (see angle α in **Fig. 7**) is defined, and the data curve is then rotated according to the defined angle α such that a slope from a minimum curve point to a maximum curve point is aligned with the axis of the first dimension X, as depicted in **Fig. 7****.**

Then, a maximum amplitude of the rotated data curve is calculated to identify the closure point of the instrument.

The end-effector is then calibrated based on the identified closure point of the surgical instrument.

According to this approach, calibration of the end-effector can be accomplished in approximately 120 microseconds.

The method of the invention may further comprise mapping a movement range for a manipulator provided on a surgeon side unit of a robotic system for use in surgery, microsurgery, or super-microsurgery, to control operation of the end-effector based on the recorded current component data and position data and the defined closure point of the instrument.

Preferably, the manipulator is a joystick.

The invention further provides a robotic system.

The robotic system is adapted for use in surgery, microsurgery or super-microsurgery.

For instance, the robotic system may be adapted to perform anastomoses.

The robotic system may be of the same kind of the system 100 of **Fig. 1****.**

The system comprises at least one robot arm, such as robot arms 112, 114 of **Figs. 1-****2.**

The system further comprises an end-effector, provided at the distal end of the at least one robot arm.

There is also a sterile drape provided in the system, said sterile drape covering the robot arm and the end-effector to maintain the sterile barrier during surgery.

The system further comprises at least one surgical instrument having a first arm and a second arm.

In particular, said surgical instrument can be adapted to switch between an open configuration and a closed configuration.

As a non-limiting example, the surgical instrument can be a hinged surgical instrument.

For instance, said surgical instrument can be a scissors-type or a forceps-type instrument.

Alternatively, said surgical instrument can be a dilator.

The system further comprises a surgical instrument adapter, providing an interface for operative connection between the at least one surgical instrument and the end effector.

Still further, the system comprises a processing means, said processing means being configured and adapted to run a dedicated non-volatile computer-readable medium to implement and/or process and/or perform the method steps defined above.

Still further, the present invention provides a computer-readable non-volatile storage medium comprising computer-readable instructions that, when executed by a processing means, causes said processing means to implement and/or process and/or perform the method steps defined above.

In connection with the above disclosure, the following aspects are explicitly disclosed:
**Aspect 1:** A surgical instrument adapter configured to transmit a movement of a tip portion of first and second movable fingers of an end-effector to at least one surgical instrument to be operated through the robot arm, characterized in that it comprises:
   a first movable arm;
   a second movable arm, and
   a base portion,

   wherein the first movable arm and the second movable arm are hingedly connected through said base portion,
   wherein the base portion the surgical instrument adapter comprises a first locking portion, comprising or defining a connection means for detachably connecting the surgical instrument adapter to the end-effector,
   wherein said first locking portion is configured to switch between:
      a first, locked state where the first locking portion is configured to secure the surgical instrument adapter to the end-effector, and
      a second, unlocked state for removing the surgical instrument adapter from the end-effector base,
   wherein the base portion the surgical instrument adapter further comprises a user interface portion, configured to be operated through the fingers of a user to switch the first locking portion between the first, locked state and the second, unlocked state, and
   wherein the surgical instrument adapter further comprises a second locking portion, comprising or defining a connection means for detachably connecting the surgical instrument adapter to the at least one surgical instrument to be manipulated trough the robot arm.
**Aspect 2:** The surgical instrument adapter according to aspect 1, wherein:
   the first movable arm comprises or defines a first grip portion at its distal end, said first grip portion being configured to detachably connect the distal end of the first movable arm to the tip portion of the first movable finger of the end-effector, and
   the second movable arm comprises or defines a second grip portion at its distal end, said second grip portion being configured to detachably connect the distal end of the second movable arm to the tip portion of the second movable finger of the end-effector.
**Aspect 3:** The surgical instrument adapter according to aspect 2, wherein:
   the shape of the first and second grip portions is complementary with respect to the shape of the tip portion of the first and second movable fingers of the end-effector,
   preferably wherein the first and second grip portions are configured for connection with respect to the tip of the first, respectively second movable fingers of the end-effector through snap-fitting.
**Aspect 4:** The surgical instrument adapter according to aspects 2 or 3, wherein:
   the first grip portion is made in one-piece with the first movable arm of the surgical instrument adapter, and
   the second grip portion is made in one-piece with the second movable arm of the surgical instrument adapter.
**Aspect 5:** The surgical instrument adapter according to any one of the preceding aspects, wherein the second locking portion comprises:
   a first U-shaped portion, inwardly extending from the first movable arm, preferably from the distal end of the first movable arm; and
   a second U-shaped portion, inwardly extending from the second movable arm, preferably from the distal end of the second movable arm,
   preferably wherein the first and second U-shaped portions are configured for connection with respect to the at least one surgical instrument through snap-fitting.
**Aspect 6:** The surgical instrument adapter according to aspect 5, wherein:
   the first U-shaped portion comprises a first opening and a first protruding portion, and
   the second U-shaped portion comprises a second opening and a second protruding portion,
   wherein the first protruding portion and the second opening, respectively the second protruding portion and the first opening have complementary shapes.
**Aspect 7:** The surgical instrument adapter according to any one of the preceding aspects, wherein:
   the user interface portion includes a first interface part and a second interface part, configured to be grasped and operated through the user's fingers to control a state of the first locking portion,
   wherein said first and second interface parts are configured to switch between:
      a first position, where said first and second interface parts are spaced apart from one another and the first locking portion is maintained in the first, locked state, and
      a second position, where said first and second interface parts are brought closer to one another by exerting a pressing force on said first and second interface parts, and the first locking portion is switched to the second, unlocked state,
   preferably wherein the first and second interface parts have a concave shape.
**Aspect 8:** The surgical instrument adapter according to aspect 7, wherein each of the first and second interface parts includes a distal protruding portion and/or a proximal protruding portion.
**Aspect 9:** The surgical instrument adapter according to any one of the preceding aspects, wherein the first locking portion includes a first protrusion and a second protrusion, formed on opposite sides, said first and second protrusions being configured to engage with corresponding first and second grooves formed in the end-effector base, preferably through snap-fitting.
**Aspect 10:** The surgical instrument adapter according to any one of the preceding aspects, wherein the first locking portion has a tapered shape.
**Aspect 11:** The surgical instrument adapter according to any one of the preceding aspects, wherein the first and second U-shaped portions are configured to maintain alignment between the longitudinal axis of the at least one surgical instrument and the longitudinal axis of the end-effector at the point of interaction between the surgical instrument adapter and the surgical instrument.
**Aspect 12:** The surgical instrument adapter according to any one of the preceding aspects, wherein the first locking portion is configured to maintain alignment of the surgical instrument adapter along the longitudinal axis of the at least one surgical instrument.

### Reference list

- 100: Robotic system
- 102: Base station
- 104: Base column
- 106: Display module
- 108: Suspension arm
- 110: Fork-like element
- 112: (First) surgical, microsurgical or super-microsurgical robot arm
- 114: (Second) surgical, microsurgical or super-microsurgical robot arm
- 116: Surgical instrument
- 118: Surgical instrument
- 120: Wheels

- B: Operating bed
- M: Microscope
- S1: First surgeon
- S2: Second surgeon
- P: Patient
- R: Operating room

- X: First dimension of the coordinate system
- Y: Second dimension of the coordinate system
- X=Y: Line

## Claims

1. A method of calibrating an end-effector provided at the distal end of a robot arm, the method comprising:
connecting a surgical instrument having a first arm and a second arm to the end-effector, the instrument being adapted for switching between and/or being used in an open configuration and a closed configuration;
performing an opening-closing-opening cycle of the surgical instrument;
measuring and recording force data related to (closure) force applied to the instrument, especially such as current data and position data during the opening-closing-opening cycle and /or related sensor data;
defining and recording a relationship between force data and position data during the opening-closing-opening cycle;
analyzing the relationship and determining a closing point of the surgical instrument, and
calibrating the end-effector based on the determined closing point of the surgical instrument.

2. The method of claim 1,
**characterized in that**
the method further comprises a step of noise reduction, wherein said step of noise reduction consists in or comprises smoothing the measured current component data,
preferably wherein said step of noise reduction is implemented by using a moving average.

3. The method of claim 2,
**characterized in that**
the method further comprises normalizing smoothed current component data within a unit square, to standardize analysis scale.

4. The method of claim 3,
**characterized in that**
the method further comprises:
defining a data vector in the data curve and rotating the defined vector by 45° to align a slope of the axes of the coordinate system, with X=Y with the curve;
calculating an amplitude of the rotated vector (|V|), this value corresponding to a difference (X-Y);
identifying maximum amplitude of the difference (X-Y), corresponding to the closure point of the surgical instrument, and
scaling data back to the original dimensions to locate the closure point within the actual dataset.

5. The method of claim 2,
**characterized in that**
the method further comprises:
defining an angle of rotation and rotating the data curve according to the defined angle of rotation, such that a slope from a minimum curve point to a maximum curve point is aligned with the axis of the first dimension (X), and
calculating a maximum amplitude of the rotated data curve to identify the closure point of the surgical instrument.

6. The method of any one of the preceding claims,
**characterized in that**
the method further comprises:
mapping a movement range for a manipulator provided on a surgeon side unit of a robotic system for use in surgery, microsurgery, or super-microsurgery, to control operation of the end-effector based on the recorded current component data and position data, and the defined closure point of the surgical instrument,
preferably wherein said manipulator is a joystick.

7. The method of any one of the preceding claims,
**characterized in that**
the position data for the surgical instrument are based on encoder data.

8. The method of any one of the preceding claims,
**characterized in that**
the step of calibrating the end-effector comprises defining, based on the determined closing point of the surgical instrument, one or more among the following operation parameters for the surgical instrument:
maximum opening position, and/or
maximum closure position; and/or
maximum closure force.

9. The method of any one of the preceding claims,
**characterized in that**
the current component data are quadrature current component data.

10. The method of any one of the preceding claims,
**characterized in that**
the surgical instrument is a hinged surgical instrument,
preferably wherein:
the surgical instrument is a scissors-type or a forceps-type surgical instrument, or
the surgical instrument is a dilator.

11. A robotic system for use in surgery, microsurgery or super-microsurgery, said system comprising:
at least one robot arm;
an end-effector, provided at the distal end of the at least one robot arm;
a sterile drape, covering the at least one robot arm and the end-effector;
at least one surgical instrument having a first arm and a second arm, the instrument being adapted for switching between an open configuration and a closed configuration, and
a surgical instrument adapter, providing an interface for operative connection between the at least one surgical instrument and the end effector,
wherein said robotic system further comprises a processing means that is configured and adapted to run a dedicated non-volatile computer-readable medium to implement and/or process and/or perform the method steps defined in any of claims 1 to 10.

12. The robotic system of claim 11,
**characterized in that**
the at least one surgical instrument is a hinged surgical instrument,
preferably wherein:
the at least one surgical instrument is a scissors-type or a forceps-type surgical instrument, or
the at least one surgical instrument is a dilator.

13. A computer-readable non-volatile storage medium comprising computer-readable instructions that, when executed by a processing means, causes said processing means to implement and/or process and/or perform the method steps defined in any of claims 1 to 10.
